# DEMANDE DE BREVET EUROPEEN

(11) **EP 2 586 487 A1**
(43) Date de publication de la demande: **01.05.2013**
(21) Numéro de dépôt: 12189617.9
(22) Date de dépôt: 23.10.2012
(51) Int. Cl.: A61M 39/10

(54) **Système sécurisé de transfert de liquide à usage médical**

(30) Priorité: 24.10.2011 FR 1159601
(71) Demandeur: Technoflex, 64210 Bidart (FR)
(72) Inventeur: Capitaine, François, 64600 Anglet (FR); Bonnet, Frédéric, 64100 Bayonne (FR)
(74) Mandataire: Coquel, Jean-Marc

(57) **Abrégé**

L'invention concerne un système de transfert de liquide à usage médical comportant un connecteur Luer mâle (10) et un connecteur Luer femelle (11), ledit connecteur Luer mâle (10) comportant un collier entourant au moins une partie d'une portion conique mâle (14) dont la surface externe vient s'appuyer contre la surface de raccordement conique femelle (15) dudit connecteur Luer femelle (11) lors de l'assemblage desdits connecteurs. Selon l'invention,
- le diamètre intérieur maximal à l'entrée du collier (12) du connecteur Luer mâle (10) est inférieur strictement au plus petit diamètre extérieur d'un connecteur Luer femelle (11) standard ou selon la norme ISO 594-2,
- le connecteur Luer femelle (11) comporte un collier (19) entourant au moins une partie du raccordement conique femelle (15), ce collier (19) ayant un diamètre intérieur qui est supérieur ou égal au diamètre extérieur du collier (12) du connecteur Luer mâle (10) de manière à venir s'engager et recouvrir au moins partiellement le collier (12) de ce connecteur Luer mâle (10) lors de l'assemblage desdits connecteurs Luer mâle et femelle.

## Description

La présente invention concerne le domaine des raccords Luer et en particulier un système sécurisé de transfert de solutions à usage médical.

Il est connu d'utiliser des raccords Luer pour connecter des dispositifs médicaux entre eux en vue de transférer une solution médicale par exemple d'un premier récipient vers un conduit corporel humain.

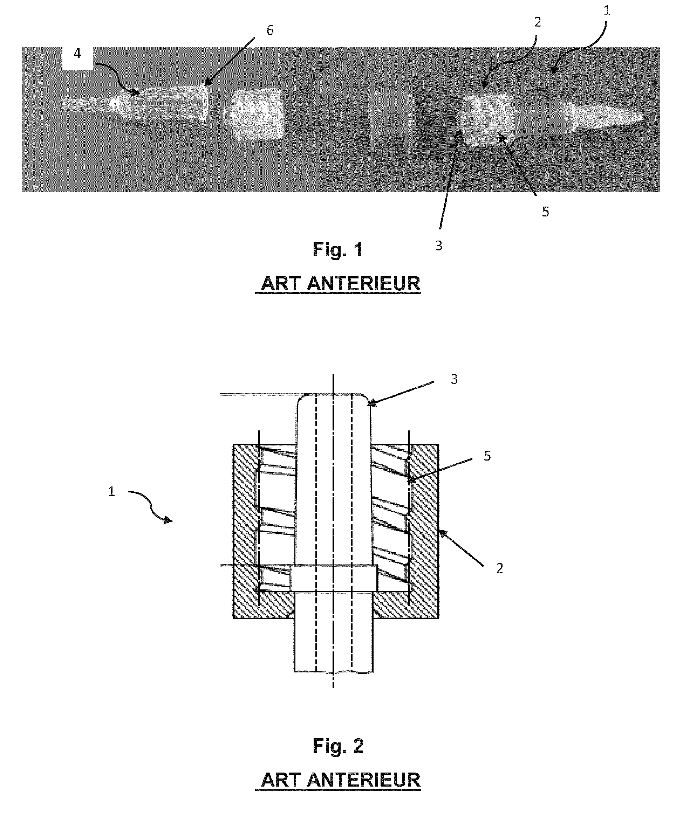

Les Figures 1 et 2 montrent des raccords Luer mâle et femelle de l'état de l'art. Un raccord Luer mâle 1 comprend ainsi un collier 2 entourant au moins une partie d'une portion conique mâle 3 dont la surface externe vient s'appuyer contre la surface de raccordement conique femelle du raccord Luer femelle 4 lors de l'assemblage de ces raccords Luer mâle 1 et femelle 4.

Le collier 2 du raccord Luer mâle 3 comporte un filetage interne 5 tandis que le raccord Luer femelle 4 comporte à son extrémité la plus large des oreilles 6 destinées à coopérer avec le filetage interne 5 du collier 2 du raccord Luer mâle 1 en vue de leur assemblage.

L'assemblage des deux raccords Luer 1, 4 est obtenu de manière simple par application par l'opérateur d'un couple de serrage. L'application de ce couple de serrage, ou mouvement de torsion, entraîne ainsi par exemple un mouvement de rotation du raccord Luer femelle 4 autour du filetage interne 5 du collier 2 du connecteur mâle.

Les raccords Luer 1, 4 ainsi engagés et en prise sont solidarisés entre eux, ce qui empêche toute désolidarisation intempestive et contamination extérieure de ces raccords Luer, tel que cela a été défini par la norme ISO 594-2 (Organisation Internationale de normalisation).

Cette dernière norme définit par ailleurs des dimensions précises pour chacun de ces raccords Luer.

Bien que ces raccords Luer 1, 4 soient faciles d'utilisation, on a toutefois récemment constaté le décès d'un patient lié à une erreur de manipulation par l'opérateur lors de l'accouplement d'un grand nombre de raccords Luer.

Notamment, lors des manipulations nécessaires en aphérèse, l'opérateur avait inversé deux lignes d'anticoagulant et sérum physiologique, lesquelles sont actuellement équipées de raccords Luer identiques.

Il existe donc un besoin urgent de faire évoluer les connecteurs de l'état de l'art de manière à éviter toute nouvelle erreur humaine de manipulation susceptible d'entrainer des risques pour la vie du patient.

La présente invention vise à pallier cet inconvénient en proposant un système de transfert de liquide à usage médical particulièrement simple dans sa conception et dans son mode opératoire, aseptique et économique, permettant un raccordement entièrement sécurisé des connecteurs Luer mâle et femelle.

A cet effet, l'invention concerne un système de transfert de liquide à usage médical comportant un connecteur Luer mâle et un connecteur Luer femelle, le connecteur Luer mâle comportant un collier entourant au moins une partie d'une portion conique mâle dont la surface externe vient s'appuyer contre la surface de raccordement conique femelle du connecteur Luer femelle lors de l'assemblage desdits connecteurs Luer mâle et femelle.

Selon l'invention,
- le collier du connecteur Luer mâle comportant un diamètre intérieur et un diamètre extérieur, le diamètre intérieur maximal à l'entrée de ce collier est inférieur strictement au plus petit diamètre extérieur d'un connecteur Luer femelle standard ou selon la norme ISO 594-2,
- le connecteur Luer femelle comporte un collier entourant au moins une partie dudit raccordement conique femelle, le collier du connecteur Luer femelle ayant un diamètre intérieur qui est supérieur ou égal au diamètre extérieur du collier du connecteur Luer mâle de manière à venir s'engager et recouvrir au moins partiellement ce collier du connecteur Luer mâle lors de l'assemblage desdits connecteurs Luer mâle et femelle.

Dans son mode de réalisation le plus général, le désengagement des connecteurs Luer mâle et femelle peut être obtenu soit application d'un couple de desserrage, soit par application d'une force de traction axiale.

Dans ce dernier cas, et à titre purement illustratif, l'assemblage des connecteurs Luer mâle et femelle est obtenu par emboitement du collier femelle sur le collier mâle du connecteur Luer mâle, le collier du connecteur Luer femelle venant recouvrir au moins partiellement le collier du connecteur Luer mâle.

Afin de renforcer l'assemblage de ces deux connecteurs, les colliers peuvent être allongés pour présenter une surface de contact plus importante et/ou la surface d'au moins un des deux colliers destinée à venir en contact avec la surface de l'autre collier peut être une surface rugueuse pour augmenter la friction entre les deux surfaces. Le collier mâle peut également présenter alternativement une ou plusieurs saillies annulaires pour empêcher la désolidarisation des deux connecteurs Luer.

En accord avec la norme ISO 594-2, le diamètre intérieur libre du collier du connecteur Luer mâle est donc au maximum de 7,82 mm de manière à refuser toute insertion par erreur d'un connecteur Luer femelle de l'état de l'art.

De préférence, le diamètre intérieur libre du collier du connecteur Luer femelle est alors au maximum de 7,90 +/- 0,05 mm.

Afin de ne pas limiter indûment la portée de son invention et pour tenir compte des évolutions éventuelles des dimensions des raccords Luer de l'état de l'art, le demandeur ne souhaite pas limiter le diamètre interne de son connecteur Luer mâle à une valeur bien définie.

Dans différents modes de réalisation particuliers de ce système, chacun ayant ses avantages particuliers et susceptibles de nombreuses combinaisons techniques possibles:
- le système comporte des moyens d'attache permettant de solidariser lesdits connecteurs Luer mâle et femelle lors de leur assemblage ou après leur assemblage, au moins une partie desdits moyens d'attache étant destinée à être placée extérieurement audit connecteur Luer mâle,

Ces moyens d'attache sont par conséquent placés à l'extérieur du volume intérieur défini entre le collier du connecteur Luer mâle et sa portion conique mâle.

A titre purement illustratif, ces moyens d'attache peuvent être rapportés sur la surface externe du collier du connecteur Luer femelle après assemblage des connecteurs Luer mâle et femelle. Il peut alors s'agir d'une bande élastique ou d'un collier de serrage tel qu'un collier en plastique de type Serflex (marque déposée).

A titre d'exemple, ce collier de serrage peut ainsi comporter une ceinture comportant des crans formant une première crémaillère et une agrafe, ladite agrafe comportant d'une part des dents d'accrochage anti-retour et d'autre part un logement pour assurer la mise en contact de cette première crémaillère avec lesdites dents d'accrochage anti-retour, lorsque ladite première crémaillère est engagée dans ladite agrafe, cette mise en contact entraînant le blocage en position dudit collier dans le sens du desserrage de ce collier.

Alternativement, ces moyens d'attache ne sont pas rapportés.

Il peut alors s'agir de manière large d'une structure de mise en prise ou d'engagement telle que celle formée par un filetage placé sur une portion au moins de la surface externe du collier du connecteur Luer mâle et un filetage correspondant, ou d'ailettes correspondantes, placés sur la surface interne du collier du connecteur Luer femelle. Le filetage ou les ailettes internes au collier du connecteur Luer femelle viennent s'engager et coopérer avec le filetage externe au collier du connecteur Luer mâle lors de l'assemblage des connecteurs Luer mâle et femelle.

Il peut encore s'agir d'oreilles placées sur la surface externe du collier du connecteur Luer mâle destinées à être reçues et maintenues par clipsage dans des logements correspondant formant saillie du collier du connecteur Luer femelle.
- la portion conique mâle comporte un canal intérieur dont le diamètre est au moins égal au diamètre du canal intérieur de la portion conique mâle du connecteur Luer mâle défini par la norme ISO 594-2,

Ainsi tout en étant plus compact et nécessitant moins de matière plastique à sa fabrication, le connecteur Luer mâle de la présente invention, présente un débit de solution médicale égal aux connecteurs de l'art antérieur.
- le collier du connecteur Luer femelle est mobile en rotation ou non autour dudit raccordement conique femelle,
- le collier du connecteur Luer mâle, ou respectivement le connecteur Luer femelle, comprenant au moins un élément détrompeur, le connecteur Luer femelle, ou respectivement le collier dudit connecteur Luer mâle, comprend au moins un organe de déblocage correspondant, ledit au moins un organe de déblocage s'engageant et coopérant avec ledit au moins un élément détrompeur pour autoriser l'assemblage desdits connecteurs Luer mâle et femelle lors de l'application d'un couple de serrage, ledit élément détrompeur interdisant tout assemblage du connecteur Luer pourvu d'un tel élément avec un connecteur Luer correspondant dépourvu dudit organe de déblocage,
- le collier du connecteur Luer femelle étant mobile en rotation, l'organe de déblocage placé sur la surface externe du raccordement conique femelle forme de plus un élément de guidage en translation de sorte que lors de l'application d'un couple de serrage, ledit collier réalise une rotation tandis que ledit raccordement conique femelle réalise un déplacement en translation,
- ledit au moins un organe de déblocage étant une rainure de guidage en translation placée sur la surface externe dudit raccordement conique femelle, ledit au moins un élément détrompeur est une saillie placée sur la surface interne du collier dudit connecteur Luer mâle, ladite saillie étant destinée à s'engager au moins en partie dans ladite rainure.

Le connecteur Luer femelle comprend au moins deux rainures de guidage en translation placées sur la surface externe dudit raccordement conique femelle, lesdites rainures étant diamétralement opposées par rapport audit raccordement conique femelle.
- le collier dudit connecteur Luer mâle comportant un filetage externe et ledit connecteur Luer femelle comprenant deux éléments détrompeur, chacun desdits éléments détrompeur est une patte flexible portant à son extrémité une ailette, ou oreille, destinée à s'engager et coopérer avec le filetage externe du collier dudit connecteur Luer mâle lors de l'application d'un couple de serrage, lesdits éléments détrompeur étant placés de manière à empêcher l'accès audit raccordement conique femelle en position de repos tout en pouvant être plaqués au moins partiellement contre la paroi interne dudit collier pour libérer l'accès audit raccordement conique femelle lors de l'assemblage desdits connecteurs Luer mâle et femelle,
- au moins un desdits connecteurs Luer mâle et femelle comporte une extrémité conique reliée de manière sécable ou non audit collier et en communication de fluide avec ce dernier.

Avantageusement, chaque connecteur peut comprendre en outre un élément d'obturation sécable pour garantir son asepsie. Cet élément d'obturation vient alors obturer l'extrémité libre de l'extrémité conique.

L'invention vise également un connecteur Luer mâle destiné à être utilisé dans le système de transfert de liquide tel que décrit précédemment, ledit connecteur Luer mâle comportant un filetage destiné à s'engager et coopérer avec le filetage correspondant ou les ailettes correspondantes d'un connecteur Luer femelle en vue de leur assemblage et une portion conique mâle dont la surface externe vient s'appuyer contre la surface de raccordement conique femelle dudit connecteur Luer femelle lors de l'assemblage desdits connecteurs Luer mâle et femelle.

Selon l'invention,
- ledit connecteur Luer mâle comprend un collier ayant une surface externe et une surface interne, ledit filetage étant placé sur au moins une portion de la surface externe dudit collier,
- le diamètre intérieur maximal à l'entrée dudit collier est inférieur strictement au plus petit diamètre extérieur d'un connecteur Luer femelle selon la norme ISO 594-2.

En accord avec la norme ISO 594-2, le diamètre intérieur libre du collier du connecteur Luer mâle est donc au maximum de 7,82 mm de manière à refuser toute insertion par erreur d'un connecteur Luer femelle de l'état de l'art.

Avantageusement, ladite portion conique mâle comporte un canal intérieur dont le diamètre est défini par la norme ISO 594-2 de manière à conserver un débit de liquide constant par rapports aux raccords Luer mâle de l'état de l'art.

De préférence, ce connecteur Luer mâle est réalisé d'un seul tenant en matière plastique.

L'invention vise encore un connecteur Luer femelle destiné à être utilisé dans le système de transfert de liquide tel que décrit précédemment, ce connecteur Luer femelle comprenant :
- un collier comportant des ailettes internes destinées à, ou un filetage interne destiné à, s'engager et coopérer avec un filetage placé sur au moins une portion de la surface externe du collier d'un connecteur Luer mâle lors de l'application d'un couple de serrage,
- ledit connecteur Luer femelle comportant un raccordement conique femelle dont la surface interne vient s'appuyer contre la surface externe de la portion conique mâle dudit connecteur Luer mâle lors de l'assemblage dudit connecteur Luer mâle et dudit connecteur Luer femelle.

De préférence, le collier de ce connecteur Luer femelle comportant des ailettes internes, le diamètre de ce collier pris à la base desdites ailettes est au moins égal, à + 0,1 mm prés, au diamètre du collier dudit connecteur Luer mâle pris entre son filetage extérieur.

L'invention concerne aussi un dispositif médical comportant au moins un récipient contenant un liquide à usage médical équipé d'un système de transfert de liquide à usage médical tel que décrit précédemment.

L'invention concerne enfin un dispositif médical comportant une seringue équipée d'un connecteur Luer mâle ou d'un connecteur Luer femelle tels que décrits précédemment.

Enfin, l'invention encore un système de transfert de liquide à usage médical comportant un connecteur Luer mâle et un connecteur Luer femelle, ledit connecteur Luer mâle comportant un collier entourant au moins une partie d'une portion conique mâle dont la surface externe vient s'appuyer contre la surface de raccordement conique femelle dudit connecteur Luer femelle lors de l'assemblage desdits connecteurs Luer mâle et femelle.

Selon l'invention,
- ledit connecteur Luer femelle comporte un collier entourant au moins une partie dudit raccordement conique femelle, ledit collier ayant un diamètre intérieur qui est supérieur ou égal au diamètre extérieur du collier dudit connecteur Luer mâle de manière à venir s'engager et recouvrir au moins partiellement ledit collier dudit connecteur Luer mâle lors de l'assemblage desdits connecteurs Luer mâle et femelle,
- le collier dudit connecteur Luer mâle, ou respectivement le connecteur Luer femelle, comprenant au moins un élément détrompeur, ledit connecteur Luer femelle, ou respectivement le collier dudit connecteur Luer mâle, comprend au moins un organe de déblocage correspondant, ledit au moins un
organe de déblocage s'engageant et coopérant avec ledit au moins un élément détrompeur pour autoriser l'assemblage desdits connecteurs Luer mâle et femelle lors de l'application d'un couple de serrage, ledit élément détrompeur interdisant tout assemblage du connecteur Luer pourvu d'un tel élément avec un connecteur Luer correspondant dépourvu dudit organe de déblocage.

Le diamètre interne du collier du connecteur Luer mâle peut ainsi être égal au diamètre du collier d'un connecteur Luer mâle standard ou selon la norme ISO 594-2.

Alors que le ou les éléments détrompeur, ou le ou les organes de déblocage, du connecteur Luer mâle sont forcément placés sur le collier, ce ou ces éléments, ou ce ou ces organes, correspondants peuvent être placés sur le collier et/ou la surface externe dudit raccordement conique femelle pour le connecteur Luer femelle.

L'invention sera décrite plus en détail en référence aux dessins annexés dans lesquels:
- la figure 1 est une vue d'un ensemble raccord Luer mâle/raccord Luer femelle selon un mode de réalisation de l'art antérieur;
- la figure 2 est une vue en coupe partielle du raccord Luer mâle de la Fig. 1,
- la figure 3 est une vue de profil d'un système de transfert de liquide à usage médical comportant un connecteur Luer mâle et un connecteur Luer femelle, selon un premier mode de réalisation de l'invention, la Fig. 3a) montrant la portion conique mâle du connecteur Luer mâle et la Fig. 3b) montrant le raccordement conique femelle du connecteur Luer femelle,
- la figure 4 est une vue de profil d'un système de transfert de liquide à usage médical comportant un connecteur Luer mâle et un connecteur Luer femelle, selon un second mode de réalisation de l'invention, la Fig. 4a) montrant le raccordement conique femelle du connecteur Luer femelle et la Fig. 3b) montrant la portion conique mâle du connecteur Luer mâle,

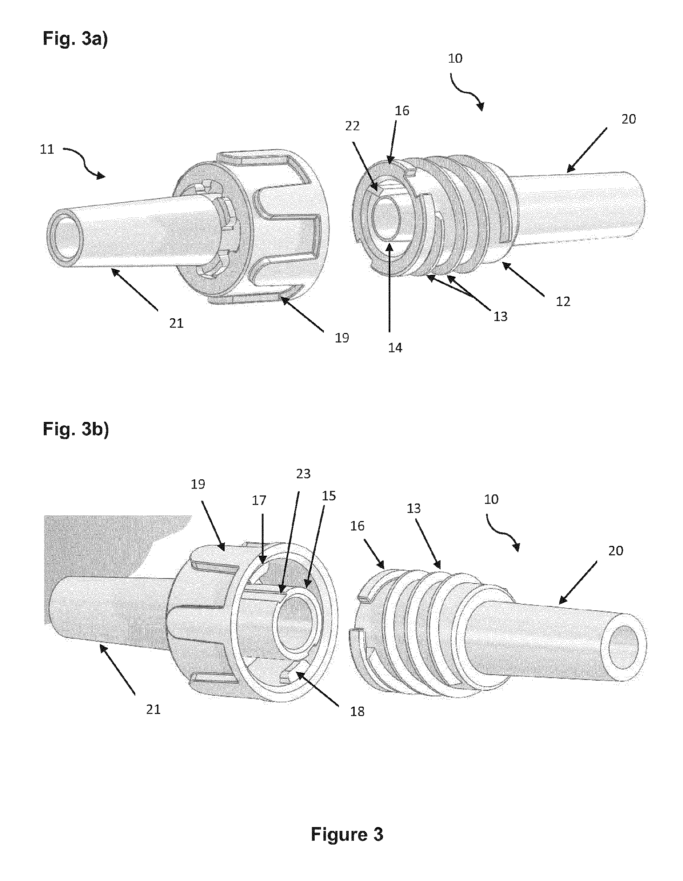

La figure 3 est une représentation schématique d'un système de transfert de liquide à usage médical selon un premier mode de réalisation de l'invention.

Ce système de transfert comporte un connecteur Luer mâle 10 et un connecteur Luer femelle 11 destinés à être assemblés pour permettre le transfert du liquide par application d'un couple de serrage.

Le connecteur Luer mâle 10 comporte un collier, ou une jupe, 12 fixe ayant une surface externe présentant un filetage 13. Ce collier 12 entoure une portion conique mâle 14 dont la surface externe vient s'appuyer contre la surface de raccordement conique femelle 15 du connecteur Luer femelle 11 lors de l'assemblage de ces deux connecteurs Luer.

Le collier 12 du connecteur Luer mâle présentant un diamètre intérieur et un diamètre extérieur, le diamètre intérieur maximal à l'entrée de ce collier, c'est-à-dire au niveau de l'extrémité de ce collier permettant l'introduction du raccordement conique femelle 15, est inférieur strictement au plus petit diamètre extérieur d'un connecteur Luer femelle standard ou selon la norme ISO 594-2. Ainsi, un opérateur devant raccorder plusieurs paires de connecteur ne peut avantageusement pas connecter par erreur un connecteur Luer femelle de l'état de l'art avec le connecteur Luer mâle de l'invention.

Conformément à la norme actuelle ISO 594-2, le diamètre intérieur libre du collier du connecteur Luer mâle est donc au maximum de 7,82 mm

Avantageusement, la portion conique mâle 14 qui présente un axe de symétrie confondu avec l'axe de symétrie du collier 12 du connecteur mâle, définit un canal intérieur pour le passage du liquide à usage médical à transférer. Le diamètre de ce canal intérieur est au moins égal au diamètre du canal intérieur de la portion conique mâle des connecteurs Luer mâle de l'état de l'art de manière à conserver un débit de solution au moins similaire, voire supérieur.

L'extrémité du collier 12 du connecteur Luer mâle 10 présente en outre une couronne, ou bourrelet, 16 non continue de manière à définir des ports d'entrée permettant d'accéder ou non au filetage externe 13 du collier du connecteur Luer mâle 10. Ces ports d'entrée ne peuvent être franchis que par des ailettes, ou oreilles, 17, 18 de forme complémentaire placée à l'intérieur du collier 19 mobile du connecteur Luer femelle, ces ailettes 17, 18 étant destinées à venir s'engager dans le filetage externe 13 du collier 12 du connecteur Luer mâle 10 pour permettre au collier 19 mobile du connecteur Luer femelle 11 d'entrer en rotation autour de ce filetage 13 lors de l'application d'un couple de serrage.

Le connecteur Luer mâle 10 est d'un seul tenant et obtenu ici par moulage par injection d'une matière thermoplastique médicalement inerte. Il peut, à titre purement illustratif, être réalisé en polyoléfine (tel que le polyéthylène, le polypropylène, un homopolymère ou un copolymère de polypropylène) ou en polyoléfine comprenant un thermoplastique élastomère (TPE) tel que le SEB (copolymère bloc Styrène, Ethylène, Butylène), le SEBS (copolymère bloc Styrène, Ethylène, Butylène, Styrène)...

Le collier 12 fixe du connecteur Luer mâle 10 est de plus relié à une extrémité conique 20 destinée à être insérée dans un conduit tel qu'une tubulure (non représenté). Cette extrémité conique 20 est reliée de manière sécable ou non au collier 12 et en communication de fluide avec le canal intérieur de la portion conique mâle 14.

Le collier 19 du connecteur Luer femelle 11 comporte pour sa part deux ailettes internes, ou oreilles intérieures, 18, 19 placées à l'opposée l'une de l'autre sur la paroi interne du collier 19 de manière à s'engager dans les ports d'entrée définis par le bourrelet 16 non continu du collier 12 du connecteur Luer mâle lors de l'accouplement des connecteurs Luer mâle 10 et femelle 11. Ces oreilles 18, 19 ayant passé ce bourrelet 16 non continu, elles peuvent alors s'engager et coopérer avec le filetage 13 placé sur la surface externe du collier 12 du connecteur Luer mâle 10 lors de l'application d'un couple de serrage.

Le collier 19 du connecteur Luer femelle 11 est mobile en rotation autour du raccordement conique femelle 15, lequel est relié à une extrémité conique 21 destinée à être insérée dans une tubulure. Le raccordement conique femelle 15 est placé de manière coaxiale avec le collier 19 du connecteur Luer femelle 11.

Par ailleurs, le collier 12 du connecteur Luer mâle comprend deux éléments détrompeur internes 22 c'est-à-dire deux éléments détrompeur placés sur la surface intérieure du collier 12 dans l'espace séparant ce collier de la portion conique mâle 14 du connecteur Luer mâle 10. Ces deux éléments détrompeur 22 autorisent l'accouplement du connecteur Luer mâle 10 uniquement avec un connecteur Luer femelle 11 comportant deux organes de déblocage 23 correspondants. Chaque organe de déblocage 23 est ainsi destiné à s'engager et coopérer avec un des éléments détrompeur 22 pour permettre l'assemblage des connecteurs Luer mâle et femelle lors de l'application d'un couple de serrage.

Les éléments détrompeur 22 sont ici des saillies dont la section droite est de forme rectangulaire et les organes de déblocage 23 sont des rainures de forme correspondante aux saillies, ces rainures étant placées sur la surface externe du raccordement conique femelle 15.

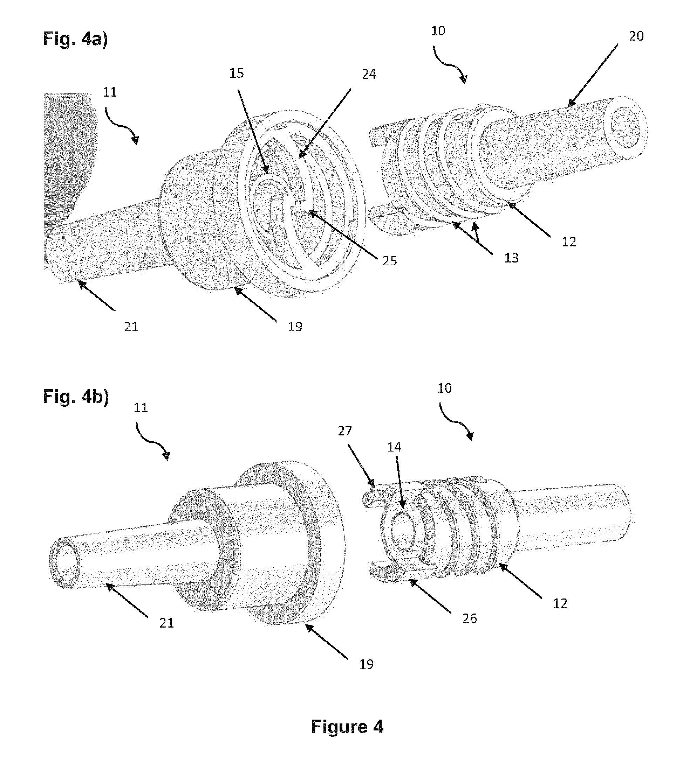

La Figure 4 est une vue de profil d'un système de transfert de liquide à usage médical comportant un connecteur Luer mâle et un connecteur Luer femelle, selon un second mode de réalisation de l'invention. Les éléments de la Figure 4 portant les mêmes références que les éléments de la Figure 3 représentent les mêmes objets, lesquels ne seront pas décrits de nouveau ci-dessous.

Le système de transfert de liquide de la Figure 4 diffère de celui décrit plus haut en ce que connecteur Luer femelle 11 comprend deux éléments détrompeur, chacun de ces éléments détrompeur étant constitué d'une patte flexible 24 portant à son extrémité une ailette, ou oreille, 25 destinée à s'engager et coopérer avec le filetage externe 13 du collier 12 du connecteur Luer mâle lors de l'application d'un couple de serrage.

Ces éléments détrompeur 24, 25 sont placés de manière à empêcher l'accès au raccordement conique femelle 15 en position de repos tout en pouvant être plaqués au moins partiellement contre la paroi interne du collier 19 fixe du connecteur Luer femelle 11 pour libérer l'accès à ce raccordement conique femelle 15 en position d'assemblage du connecteur Luer mâle et du connecteur Luer femelle. Une partie de ces éléments détrompeur 24, 25 est donc placée en avant du raccordement conique femelle pour former un écran empêchant l'accès à ce raccordement conique femelle 15.

Le connecteur Luer mâle 10 comporte pour sa part des organes de déblocage 26, 27 permettant de déverrouiller les éléments détrompeur 24, 25 du connecteur Luer femelle 11 pour mettre en contact étanche la portion conique mâle 14 et le raccordement conique femelle 15.

Ces organes de déblocage 26, 27 sont ici des projections formant saillie du collier 12 du connecteur Luer mâle 10. Ces projections 26, 27 de forme courbe sont espacées l'une de l'autre de manière à pouvoir être insérées entre lesdits éléments détrompeur 24, 25 et le collier 19 fixe du connecteur Luer femelle 11 pour venir s'engager dans ce collier 19.

Ces organes de déblocage 26, 27 viennent alors en appui contre les pattes flexibles 24 lors de l'application d'un couple de serrage de manière à entraîner la partie libre de ces pattes 24 et de la plaquer contre la paroi interne du collier 19 du connecteur Luer femelle libérant ainsi l'accès au raccordement conique femelle 15.

Le collier 19 du connecteur Luer femelle 11 ayant une forme cylindrique creuse, ces pattes flexibles 14 sont arrondies de manière à pouvoir être plaquées intégralement contre la paroi interne du collier 19 et libérer ainsi entièrement l'accès au raccordement conique femelle 15.

## Revendications

1. Système de transfert de liquide à usage médical comportant un connecteur Luer mâle (10) et un connecteur Luer femelle (11), ledit connecteur Luer mâle (10) comportant un collier (12) entourant au moins une partie d'une portion conique mâle (14) dont la surface externe vient s'appuyer contre la surface de raccordement conique femelle (15) dudit connecteur Luer femelle (11) lors de l'assemblage desdits connecteurs Luer mâle et femelle, **caractérisé en ce que**
- le collier (12) dudit connecteur Luer mâle (10) comportant un diamètre intérieur et un diamètre extérieur, le diamètre intérieur maximal à l'entrée dudit collier (12) est inférieur strictement au plus petit diamètre extérieur d'un connecteur Luer femelle (11) selon la norme ISO 594-2,
- ledit connecteur Luer femelle (11) comporte un collier (19) entourant au moins une partie dudit raccordement conique femelle (15), ledit collier ayant un diamètre intérieur qui est supérieur ou égal au diamètre extérieur du collier (12) dudit connecteur Luer mâle (10) de manière à venir s'engager et recouvrir au moins partiellement ledit collier (12) dudit connecteur Luer mâle (10) lors de l'assemblage desdits connecteurs Luer mâle et femelle.

2. Système selon la revendication 1, **caractérisé en ce qu'il** comporte des moyens d'attache (12, 13, 16, 17) permettant de solidariser lesdits connecteurs Luer mâle (10) et femelle (11) lors de leur assemblage ou après leur assemblage, au moins une partie desdits moyens d'attache étant destinée à être placée extérieurement audit connecteur Luer mâle (10).

3. Système selon la revendication 1 ou 2, **caractérisé en ce que** ladite portion conique mâle (14) comporte un canal intérieur dont le diamètre est au moins égal au diamètre du canal intérieur de la portion conique mâle (14) du connecteur Luer mâle (10) défini par la norme ISO 594-2.

4. Système selon l'une quelconque des revendications 1 à 3, **caractérisé en ce que** le collier dudit connecteur Luer femelle (11) est mobile en rotation autour dudit raccordement conique femelle (15).

5. Système selon l'une quelconque des revendications 1 à 4, **caractérisé en ce que** le collier (12) dudit connecteur Luer mâle (10), ou respectivement le connecteur Luer femelle (11), comprenant au moins un élément détrompeur (22, 24, 25), ledit connecteur Luer femelle (11), ou respectivement le collier (12) dudit connecteur Luer mâle (10), comprend au moins un organe de déblocage (23, 26, 27) correspondant, ledit au moins un organe de déblocage (23, 26, 27) s'engageant et coopérant avec ledit au moins un élément détrompeur (22, 24, 25)pour autoriser l'assemblage desdits connecteurs Luer mâle (10) et femelle (11) lors de l'application d'un couple de serrage, ledit au moins un élément détrompeur (22, 24, 25) interdisant tout assemblage du connecteur Luer pourvu d'au moins un tel élément avec un connecteur Luer correspondant dépourvu dudit au moins un organe de déblocage (23, 26, 27).

6. Système selon la revendication 5, **caractérisé en ce que** le collier (19) dudit connecteur Luer femelle (11) étant mobile en rotation, ledit organe de déblocage placé sur la surface externe du raccordement conique femelle (15) forme de plus un élément de guidage (23) en translation de sorte que lors de l'application d'un couple de serrage, ledit collier (19) réalise une rotation tandis que ledit raccordement conique femelle (15) réalise un déplacement en translation.

7. Système selon la revendication 6, **caractérisé en ce que** ledit au moins un organe de déblocage étant une rainure de guidage (23) en translation placée sur la surface externe dudit raccordement conique femelle (15), ledit au moins un élément détrompeur (22) est une saillie placée sur la surface interne du collier dudit connecteur Luer mâle (10), ladite saillie étant destinée à s'engager au moins en partie dans ladite rainure.

8. Système selon la revendication 7, **caractérisé en ce qu'il** comprend au moins deux rainures de guidage (23) en translation placées sur la surface externe dudit raccordement conique femelle (15), lesdites rainures (23) étant diamétralement opposées par rapport audit raccordement conique femelle (15).

9. Système selon la revendication 5, **caractérisé en ce que** le collier (12) dudit connecteur Luer mâle (10) comportant un filetage externe (13) et ledit connecteur Luer femelle (11) comprenant deux éléments détrompeurs, chacun desdits éléments détrompeurs est une patte (24) flexible portant à son extrémité une oreille (25) destinée à s'engager et coopérer avec le filetage externe (13) du collier dudit connecteur Luer mâle (10) lors de l'application d'un couple de serrage, lesdits éléments détrompeurs (24, 25) étant placés de manière à empêcher l'accès audit raccordement conique femelle (15) en position de repos tout en pouvant être plaqués au moins partiellement contre la paroi interne dudit collier (19) pour libérer l'accès audit raccordement conique femelle (15) lors de l'assemblage desdits connecteurs Luer mâle et femelle.

10. Système selon la revendication 9, **caractérisé en ce que** lesdits organes de déblocage sont des projections (26, 27) formant saillie du collier (12) dudit connecteur Luer mâle (10) en étant espacés l'un de l'autre de manière à pouvoir être insérés entre lesdits éléments détrompeur (24, 25) pour s'engager dans le collier (19) dudit connecteur Luer femelle (11), lesdits organes de déblocage venant alors en appui contre lesdites pattes (24) flexibles lors de l'application d'un couple de serrage de manière à plaquer au moins partiellement lesdites pattes (24) contre la surface interne dudit collier (19) libérant ainsi l'accès audit raccordement conique femelle (15) dudit connecteur Luer femelle (11).

11. Système selon la revendication 9 ou 10, **caractérisé en ce que** le collier dudit connecteur Luer femelle (11) ayant une forme cylindrique creuse, lesdites pattes (24) flexibles sont arrondies de manière à pouvoir être plaquées intégralement contre la paroi interne dudit collier.

12. Système selon l'une quelconque des revendications 1 à 11, **caractérisé en ce qu'**au moins un desdits connecteurs Luer mâle et femelle comporte une extrémité conique (20, 21) reliée de manière sécable ou non audit collier et en communication de fluide avec ce dernier.

13. Connecteur Luer mâle (10) destiné à être utilisé dans le système de transfert de liquide selon l'une quelconque des revendications 1 à 12, ledit connecteur Luer mâle (10) comportant un filetage (12) destiné à s'engager et coopérer avec le filetage correspondant ou les ailettes correspondantes d'un connecteur Luer femelle (11) en vue de leur assemblage et une portion conique mâle (14) dont la surface externe vient s'appuyer contre la surface de raccordement conique femelle (15) dudit connecteur Luer femelle (11) lors de l'assemblage desdits connecteurs Luer mâle et femelle, **caractérisé en ce que**
- ledit connecteur Luer mâle (10) comprend un collier (12) ayant une surface externe et une surface interne, ledit filetage (13) étant placé sur au moins une portion de la surface externe dudit collier (12),
- le diamètre intérieur maximal à l'entrée dudit collier (12) est inférieur strictement au plus petit diamètre extérieur d'un connecteur Luer femelle (11) selon la norme ISO 594-2.

14. Connecteur selon la revendication 13, **caractérisé en ce qu'il** est réalisé d'un seul tenant en matière plastique.

15. Connecteur Luer femelle (11) destiné à être utilisé dans le système de transfert de liquide selon l'une quelconque des revendications 1 à 12, ledit connecteur Luer femelle (11) comprenant :
- un collier (19) comportant des ailettes (18) internes destinées à, ou un filetage interne destiné à, s'engager et coopérer avec un filetage (13) placé sur au moins une portion de la surface externe du collier (12) d'un connecteur Luer mâle (10) lors de l'application d'un couple de serrage,
- ledit connecteur Luer femelle (11) comportant un raccordement conique femelle (15) dont la surface interne vient s'appuyer contre la surface externe de la portion conique mâle (14) dudit connecteur Luer mâle (10) lors de l'assemblage dudit connecteur Luer mâle (10) et dudit connecteur Luer femelle (11).

16. Connecteur selon la revendication 15, **caractérisé en ce que** ledit collier (19) comportant des ailettes internes, le diamètre dudit collier pris à la base desdites ailettes est au moins égal, à + 0,1 mm prés, au diamètre du collier (12) dudit connecteur Luer mâle (10) considéré entre son filetage extérieur (13).

17. Dispositif médical comportant au moins un récipient contenant un liquide à usage médical équipé d'un système de transfert de liquide à usage médical selon l'une quelconque des revendications 1 à 12.
